# EUROPEAN PATENT SPECIFICATION

(11) **EP 4 185 365 B1**
(45) Date of publication and mention of the grant of the patent: **08.04.2026**
(21) Application number: 21758551.2
(22) Date of filing: 30.07.2021
(51) Int. Cl.: A61M 39/24, A61M 39/10

(54) **VALVE CONFIGURATIONS FOR A TUNABLE VALVE**
VENTILANORDNUNGEN FÜR EIN ABSTIMMBARES VENTIL
CONFIGURATIONS DE VANNE POUR UNE VANNE RÉGLABLE

(30) Priority: 31.07.2020 US 202063059652 P
(43) Date of publication of application: 31.05.2023
(73) Proprietor: Bard Access Systems, Inc., Salt Lake City, UT 84116 (US)
(72) Inventor: BLANCHARD, Daniel, B., Bountiful, UT 84010 (US)
(74) Representative: Hoffmann Eitle
(86) International application number: PCT/US2021/044061
(87) International publication number: WO 2022/026920

(56) References cited:
- US-A1- 2009 177 187
- US-A1- 2013 324 943
- US-A1- 2014 228 776

## Description

### BACKGROUND

Catheters, such as vascular catheters, can include valves which are configured to control the infusion and aspiration of fluids therethrough. These valves can be manufactured to various specifications, including various ranges of fluid pressures for crack pressures, maintenance pressures, lumen clearance, or the like for either the infusion flow direction or the aspiration flow direction. "Crack pressure" being the amount of fluid pressure required to open the valve and "maintenance pressure" being the amount of fluid pressure required to maintain the valve in an open configuration.

Current valve designs result in inconsistent specifications resulting in hundreds of thousands of dollars in scrap costs per year. For example, valves currently produced define a limited range of target specifications but a broad range of tolerances within the intended specifications. As such, two seemingly identical valves can present very different crack pressures, maintenance pressures, etc. when in use. This can present problems to the user, since these differences may mask the presence of a thrombosis or similar obstruction. Alternatively, the catheter system may be unnecessarily removed and discarded on the misinterpretation that an obstruction is present. Further problems exist with blood hemolysis, where a pressure drop across the valve during use cause damage to the cells. In addition, certain valve design can create fluid flow dead zones leading to incomplete clearance of the valve. US 2014/0228776 A1 refers to an intravascular valve assembly having a valve case and a flexible pressure-actuated flow control valve.
US 2013/0324943 A1 refers to a valve for controlling material flow through a catheter.
US 2009/0177187 A1 refers to a pressure actuated valve.

Embodiments disclosed herein are directed to valves configured to overcome the aforementioned problems by providing highly tunable specifications. These tunable specifications allow a wide range of target pressures to be provided and still achieve low tolerance variations within the target pressure, leading to more precise valve specifications. The specifications can then be optimized to mitigate blood hemolysis and to increase turbulent flow to improve lumen clearance. Further these specifications can be easily modified during the manufacturing process leading to improved manufacturing efficiency and associated cost savings.

### SUMMARY

The present invention is defined by the valved connector according to independent claim 1. Preferred optional features are recited in the dependent claims.

In some embodiments, the valved connector further includes a center slit and a side slit each extending parallel to the lateral axis, the side slit disposed in an off-set relationship along the transverse axis from the center slit. The side slit extends through the valve from the proximal face to the distal face at an angle relative to a longitudinal axis. A first side slit is angled in a first direction relative to the longitudinal axis and a second side slit is angled in a second direction opposite the first direction, relative to the longitudinal axis. The center slit opens during both infusion and aspiration and the side slit opens only during infusion. A crack pressure of the slit is greater than a maintenance pressure of the slit. One of the proximal face or the distal face of the valve includes a recess encircling a portion of the slit.

In some embodiments, the connector body includes a proximal housing piece defining a first lumen and a distal housing piece defining a second lumen, and wherein a portion of the valve is retained between the proximal housing piece and the second housing piece to control a fluid flow between the first lumen and the second lumen. A portion of the first lumen defines a reduced cross-sectional area to modify an aspiration crack pressure. A portion of the first lumen defines one of an oval shape, an oblong shape or a cross shape to direct a fluid flow towards the slit. A radius of curvature of the lateral axis can vary between d=0.5z and d=4z, where d is a midpoint distance from a linear axis and z is a longitudinal thickness of the valve.

Also disclosed is a method of manufacturing a valved connector including, forming a proximal housing piece including a first lumen and a distal engagement surface, forming a distal housing piece including a second lumen and a proximal engagement surface, forming a valve including a proximal face and a distal face and a slit extending therebetween, one of the proximal face or the distal face defining an oval shape, a lateral axis of the oval proximal housing piece and the distal housing piece to control a fluid flow between the first lumen and the second lumen, constraining the lateral axis of the valve to a curved shape to provide a convex shape to the proximal face, and attaching the distal engagement surface with the proximal engagement surface.

In some embodiments, a radius of curvature of the lateral axis can vary between d=0.5z and d=4z, where d is a midpoint distance from a linear axis and z is a longitudinal thickness of the valve between the proximal face and the distal face. The valve further includes a center slit and a side slit each extending parallel to the lateral axis and extending from the proximal face to the distal face, the side slit disposed in an off-set relationship along the transverse axis from the center slit. The side slit extends through the valve from a proximal face to a distal face at an angle relative to a longitudinal axis. A first side slit is angled in a first direction relative to the longitudinal axis and a second side slit is angled in a second direction opposite the first direction, relative to longitudinal axis.

In some embodiments, the center slit opens during both infusion and aspiration and the side slit opens only during infusion. A crack pressure of the slit is greater than a maintenance pressure thereof. One of the proximal face or the distal face of the valve includes a recess encircling a portion of the slit. A portion of the first lumen defines a reduced cross-sectional area to modify an aspiration crack pressure.

These and other features of the concepts provided herein will become more apparent to those of skill in the art in view of the accompanying drawings and the following description, which describe particular embodiments of such concepts in greater detail.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope which is defined by the appended claims. Example embodiments of the invention will be described and explained with additional specificity and detail through the use of the accompanying drawings in which:
FIG. 1A illustrates a perspective view of an exemplary catheter system including a valved connector, in accordance with some embodiments.
FIG. 1B illustrates a perspective view of the valved connector of FIG. 1A, in accordance with some embodiments.
FIGS. 1C-1D illustrate exemplary flow directions of a valve of the valved connector of FIG. 1B, in accordance with some embodiments.
FIG. 2A illustrates a proximal end view of a valve, in accordance with some embodiments.
FIG. 2B illustrates a transverse axis cross-sectional view of a valve, in accordance with some embodiments.
FIG. 2C illustrates a lateral axis cross-sectional view of a valve, in accordance with some embodiments.
FIG. 2D illustrates various exemplary radii of curvature for a lateral axis of the valve of FIG. 2C, in accordance with some embodiments.
FIG. 3A illustrates a distal end view of a valve, in accordance with some embodiments.
FIG. 3B illustrates a transverse axis cross-sectional view of the valve of FIG. 3A, in accordance with some embodiments.
FIG. 4A illustrates a proximal end view of a valve, in accordance with some embodiments.
FIG. 4B illustrates a transverse axis cross-sectional view of the valve of FIG. 4A, in accordance with some embodiments.
FIGS. 5A-5C illustrate distal end views of various embodiments of a valve, in accordance with some embodiments.
FIG. 6A illustrates a cross-sectional view of a connector body including a valve, in accordance with some embodiments.
FIGS. 6B-6D illustrate a proximal end view of various embodiments of a connector body including a valve, in accordance with some embodiments.

### DETAILED DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

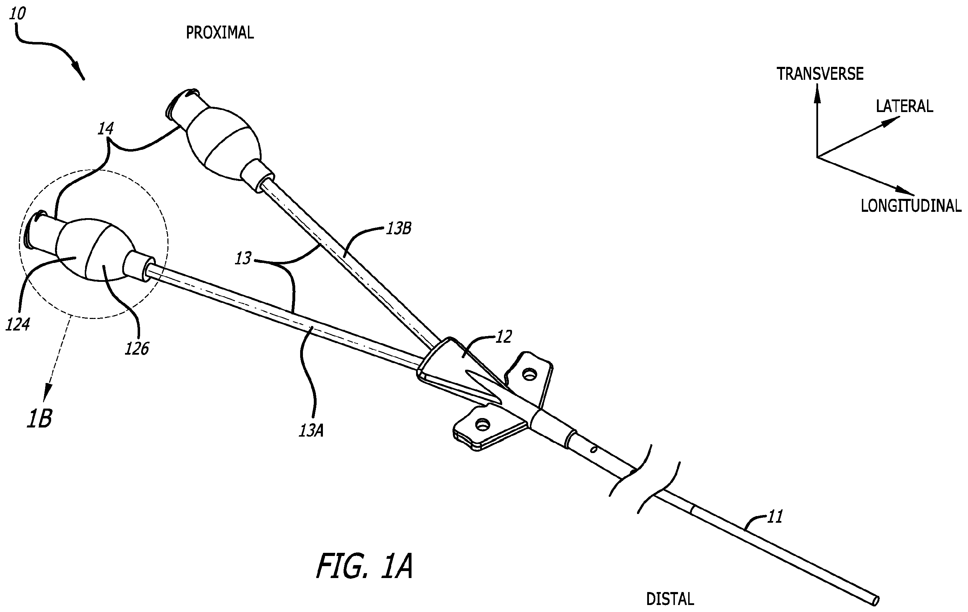

To assist in the description of embodiments described herein, as shown in FIGS. 1A-1B, a longitudinal axis extends substantially parallel to an axial length of the connector 20. A lateral axis extends normal to the longitudinal axis, and a transverse axis extends normal to both the longitudinal and lateral axes. As used herein, the term "crack pressure" is the amount of fluid pressure, or force, required to transition a valve from a closed configuration to an open configuration. As used herein, the term "maintenance pressure" is the amount of fluid pressure required to maintain the valve in an open configuration.

Unless defined otherwise, all technical and scientific terms used herein have the same meaning as commonly understood by those of ordinary skill in the art.

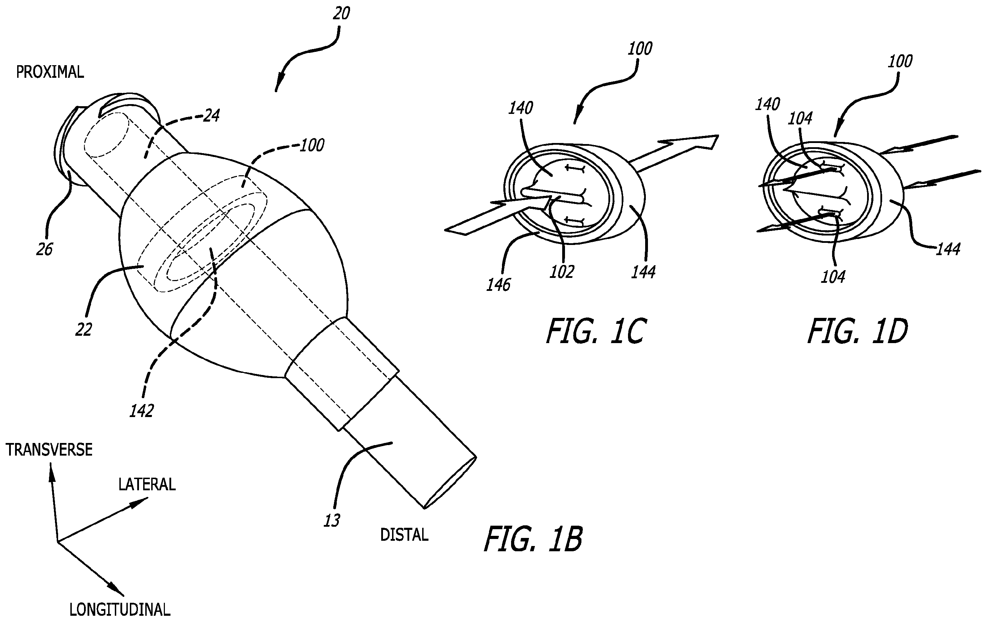

FIG. 1 shows an exemplary catheter system ("catheter") 10. The exemplary catheter 10 can be a dialysis catheter. However, it will be appreciated that embodiments disclosed herein can be used with any catheter or medical device that include valves, such as a Peripherally Inserted Central Catheter ("PICC"), Central Venous Catheters ("CVC"), intravenous ("IV") catheters, drainage catheters, midline catheters, introducer sets, port access systems, urinary catheter systems, or the like, without limitation.

The catheter 10 generally includes a catheter body 11, defining one or more lumen and supported by a catheter hub 12 coupled to a proximal end thereof. The catheter system 10 can further include an extension leg 13 extending proximally from the hub 12. The extension leg 13 can define an extension leg lumen that is in fluid communication with a lumen of the catheter body 11. It will be appreciated that the catheter system 10 can include two or more extension legs, for example a first extension leg 13A and a second extension leg 13B, each of which are in communication with a lumen of the catheter body 11, e.g. a first extension leg 13A communicates with a first lumen, and a second extension leg 13B communicates with a second lumen. The extension leg 13 can further include a connector 20 disposed at a proximal end thereof.

FIG 1B shows further details of the connector 20, in an embodiment, the connector 20 includes a body 22 that defines a lumen 24. The lumen 24 extends axially and provides fluid communication between a coupler 26, disposed at a proximal end of the connector body 22, and the extension leg 13 disposed a distal end of the connector body 22. The coupler 26 can include a luer lock, spin nut, twist lock, or similar coupling structure configured to secure a medical line, syringe, introducer, or similar device, to the connector 20.

In an embodiment, the connector 20 can include a valve 100 configured to control a fluid flow through the connector lumen 24. In an embodiment, the valve 100 can be a slit valve, however other types of valve including flap valve, duck bill valve, bileaflet, combinations thereof, or the like, are also contemplated. In an embodiment, the valve 100 can be formed of a pliable material. Exemplary pliable materials can include a polymer, elastomer, rubber, silicone, or similar suitable material, as described in more detail herein.

Embodiments of the valve 100 can provide precise crack pressures and precise maintenance pressures, with associated low tolerance ranges. This allows manufacturers to tune the valve to operate under precise flow rates. Further manufacturers can tune an increased difference between crack pressure and maintenance pressure to mitigate blood hemolysis. Further still, embodiments can provide increased turbulent flow for improved connector lumen clearance.

As shown in FIGS. 1C-2C, the valve 100 can include a proximal face 140 and a distal face 142 each extending perpendicular to the longitudinal axis, and include a side surface extending between the proximal face 140 and the distal face 142, substantially parallel to the longitudinal axis. In an embodiment, one of the proximal face 140 or the distal face 142 of the valve 100 defines a substantially oval shape. As shown in FIGS. 1C-1D, the oval shape can include a widest diameter extending along a lateral axis 106 of the valve 100, and a shortest diameter extending along a transverse axis 108 of the valve 100. However, it will be appreciated that the oval shape can also be oriented with the widest diameter extending along a transverse axis and the shortest diameter extending along the lateral axis. It will also be appreciated that the valve 100 can include other general cross-sectional shapes such as circular, hexagonal, polygonal, or similar closed curve regular or irregular polygonal shapes, or the like.

In an embodiment, one of the proximal face 140 or the distal face 142 can include a rim 146 extending annularly about a perimeter of the proximal face 140 or the distal face 142. In an embodiment, the rim 148 can be configured to engage the connector body to secure the valve 100 therein. In an embodiment, one of the proximal face 140 or the distal face 142 can include various surface configurations, such as domed, flat, half-dome, recessed, combinations thereof, or the like, as described in more detail herein.

In an embodiment, the valve 100 can include one or more slits extending from the proximal surface 140 to the distal surface 142 and configured to control a fluid flow therethrough. For example, as shown in FIGS. 1C-1D, a center slit 102 can be configured to allow a fluid flow in a first direction (e.g. infusion) and a side slit 104 can be configured to allow fluid flow in a second direction, opposite the first, (e.g. aspiration). In an embodiment, one of the center slit 102 or the side slit 104 can be configured to allow a fluid flow in both the first direction and the second direction. For example, the center slit 102 may open (actuate) during both infusion and aspiration and the side slits 104A, 104B may open during infusion only. Advantageously, this can allow for a greater fluid flow during infusion to facilitate fluid clearance from the valve 100. However, it will be appreciated that other combinations of infusion/aspiration slit actuation are also contemplated.

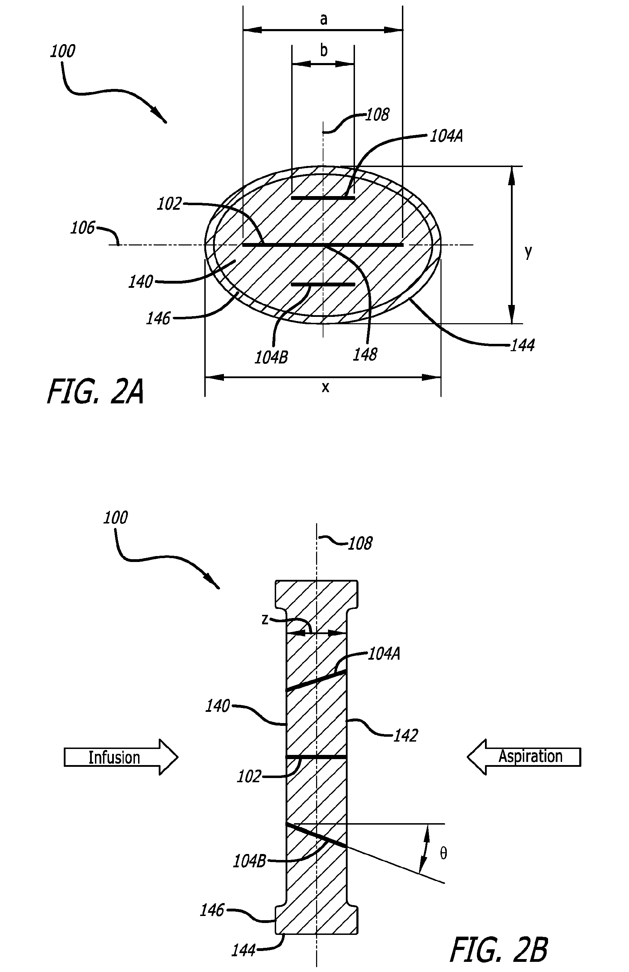

FIGS. 2A-2C show further details of the valve 100. FIG. 2A illustrates a proximal end view of the valve 100. FIG. 2B illustrates a transverse axis 108 cross sectional view of the valve 100. FIG. 1C illustrates a lateral axis 106 cross sectional view of the valve 100. As shown in FIG. 2A, in an embodiment, one or more of the center slit 102 or side slit 104 can extend parallel to the lateral axis 106. However, it will be appreciated that one of the center slit 102 or side slit 104, can extend parallel to the transverse axis 108, or at an angle relative to the lateral axis 106 and the transverse axis 108.

As noted, one of the proximal face 140 or the distal face 142 of the valve 100 can define a substantially oval shape including a lateral axis 106 defining a longest diameter (*x*), and a transverse axis 108 defining a shortest diameter (*y*). In an embodiment, the lateral axis 106 of the valve 100 can define a longest diameter (*x*) of between 0.25 in. and 0.5 in (1 in equals to 2.54 cm). In an embodiment, the transverse axis 108 of the valve 100 can define a shortest diameter (*y*) of between 0.15 in. and 0.3 in. However, it will be appreciated that greater or lesser dimensions of the longest diameter (*x*) and the shortest diameter (*y*) are also contemplated. In an embodiment, the longest diameter (*x*) extends perpendicular to the shortest diameter (*y*), although other angles are contemplated where cross-sectional shapes can define irregular or asymmetrical shapes.

In an embodiment, the valve 100 may include a center slit 102 and a first side slit 104A and a second side slit 104B. In an embodiment, the center slit 102 can extend through a cross-sectional mid-point 148 of the proximal face 144 and the side slits 104A, 104B can be off-set from the center slit 102 along the transverse axis 108. However, other configurations of slits 102, 104 are also contemplated, as discussed in more detail herein. The center slit 102 can extend parallel to the lateral axis 106 to define a first slit length (*a*). The side slit(s) 104A, 104B can also extend parallel to the lateral axis 106 and define a second slit length (*b*). In an embodiment, the second slit length (*b*) can be less than the first slit length (*a*). In an embodiment, the first slit length (*a*) can define a length of between 50%-90% of the lateral axis diameter (x). In an embodiment, the second slit length (*b*) can define a length of between 25%-40% of the lateral axis diameter (x). However, it will be appreciated that greater or lesser dimensions of first slit length (*a*) and second slit length (*b*) are also contemplated.

In an embodiment, a difference in the length of the slit 102, 104, can modify the crack pressure of the slit(s) 102, 104. For example, a relatively longer slit length can provide a relatively lower crack pressure, whereas a relatively shorter slit length can provide a relatively higher crack pressure. Exemplary crack pressures can include 0.4 psi for infusion and 3 psi for aspiration (1 psi equals 6.895 kPa). However, greater or lesser crack pressures for both infusion and aspiration are also contemplated to fall within the scope of the present invention. In an embodiment, the valve 100 defines a thickness (z) extending along a longitudinal axis between a proximal face 140 and a distal face. In an embodiment, modifying a thickness (z) of the valve 100 can modify a crack pressure of one or more slits 102, 104. For example, a relatively smaller overall thickness (*z*) of the valve 100 can provide a relatively lower crack pressure.

As shown in FIG. 2B, in an embodiment, one or more of the center slit 102 or the side slits 104 can extend through the valve 100 from the proximal face 140 to the distal face 142 parallel to the longitudinal axis. However, it will be appreciated that one of the center slit 102 or side slit 104, can also extend through the valve 100 at an angle (*θ*) with respect to the longitudinal axis. In an embodiment, the angle (*θ*) of the slide slits 104A, 104B can be between 5° and 85°. In an embodiment, the angle (*θ*) of the slide slits 104A, 104B can be between 15° and 35°. In an embodiment, the angle (*θ*) of the side slits 104 can be oriented in the same direction. In an embodiment, the angle (*θ*) of the side slits 104 can be oriented in opposing directions. For example, a first side slit 104A can be angled at + 15° from the longitudinal axis and a second side slit 104B can be angled at -15° from the longitudinal axis. In an embodiment, the angle (*θ*) of the side slits 104 can be the same or different. Advantageously, the angle (*θ*) of the side slits 104 can facilitate selective actuation depending on the direction of fluid flow through the valve. For example, the angle (*θ*) of the side slits 104 can facilitate opening during infusion while remaining closed during aspiration. In an embodiment, the slits 104A, 104B can be angled to facilitate opening during aspiration while remaining closed during infusion.

In an embodiment, the lumen 24 of the connector 20 can be cleared by applying a high fluid flow therethrough. More effective clearance of the connector lumen 24 can be achieved with an increase in turbulent flow therethrough. Further, clearing the lumen 24 may be preferably performed during infusion. Advantageously, an increased number of slits, e.g. center slit 102 or side slits 104, can result in an increased turbulent flow and provide a more effective clearance of the connector lumen 24 during either infusion or aspiration. Further, slits disposed at different angles relative to either the lateral axis 106 or the longitudinal axis can further increase turbulent flow and further improve clearance efficiency.

In an unclaimed embodiment, the valve 100 can define a planar shape extending linearly through both the lateral axis 106 and the transverse axis 108. In an embodiment, one of the lateral axis 106 or the transverse axis 108 of the valve 100 can define a curvilinear shape that defines a radius of curvature (r), as described in more detail herein. In an unclaimed embodiment, both the lateral axis 106 and the transverse axis 108 can define a curved shape. In an unclaimed embodiment, the lateral axis 106 and the transverse axis 108 can curve in the same direction to provide a substantially dome shaped valve 100. In an unclaimed embodiment, the lateral axis 106 and the transverse axis 108 can curve in opposite directions to provide a substantially hyperbolic shaped valve 100.

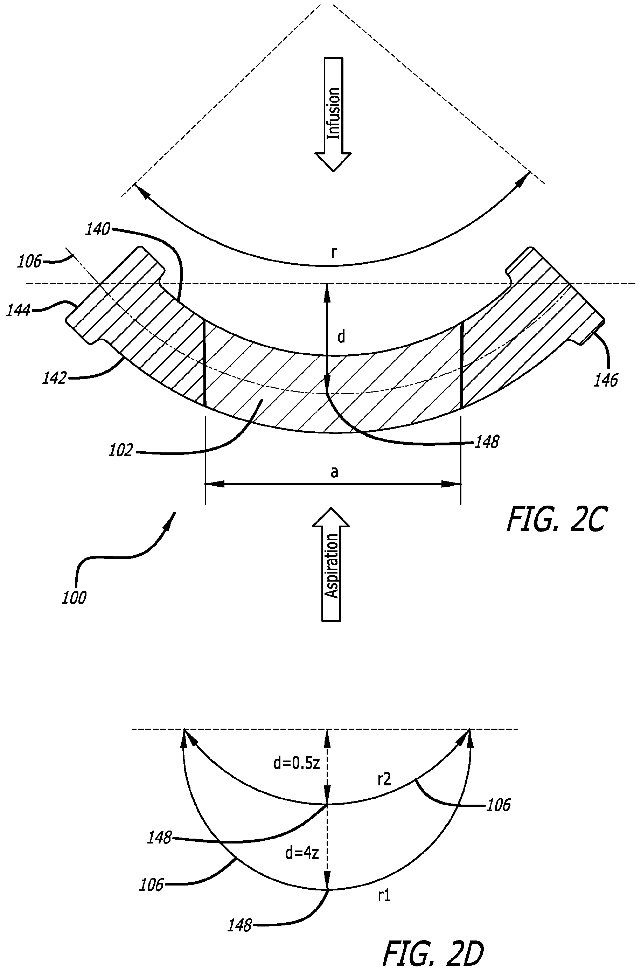

In an embodiment in accordance with the invention, as shown in FIG. 2B, the valve 100 extends linearly through the transverse axis 108 and, as shown in FIG. 2C, the valve defines a curved shape through the lateral axis 106. Advantageously, the curved lateral axis 106 can bias the valve 100 to actuate at higher crack pressure during a first fluid flow direction, and a lower crack pressure in a second direction, opposite the first. For example, as shown in FIG. 2C, the valve 100 can be curved to provide a concave proximal face 140 and a convex distal face 142. As such, the valve 100 can provide a relatively low infusion crack pressure and a relatively high aspiration crack pressure. In an embodiment, the valve 100 can be curved to provide a convex proximal face 140 and a concave distal face 142. As such, the valve 100 can provide a relatively high infusion crack pressure and a relatively low aspiration crack pressure.

In an embodiment, the curved profile of the valve 100 can provide an increased crack pressure while the maintenance pressure remains the same. As such, modifying the curved profile of the valve 100 can modify the difference between the crack pressure and the maintenance pressure, which can improve the blood hemolysis characteristics of the valve 100. For example, a relatively high crack pressure maintains a secure seal when no flow is induced. However, a relatively low maintenance pressure reduces the pressure drop across the valve when a flow is induced, which in turn mitigates damage to blood cell, i.e. blood hemolysis.

In an embodiment, a radius of curvature (r) of the lateral axis 106 can be modified to change the crack pressure of one or more of the central slit 102 or the side slits 104A, 104B. For example, as shown in FIG. 2D, a smaller radius of curvature (*r1*), which may be considered a "tighter" arc, may provide a higher aspiration crack pressure of the central slit 102. By contrast, a larger radius of curvature (r2), which may be considered a "wider" arc, provides a lower aspiration crack pressure of the central slit 102.

As shown in FIGS. 2C-2D, in an embodiment, a radius of curvature (r) can be modified to change a distance of mid-point 148 from a linear axis, termed "mid-point distance" (d). As such the radius of curvature (*r*) can be modified such that a midpoint distance (d) varies between 0.5z and 4z, where z is the overall thickness of the valve 100. In an embodiment, the radius of curvature (r) along the lateral axis 106 can be modified to provide an aspiration crack pressure of between 1psi and 9 psi. In an embodiment, the radius of curvature (r) along the lateral axis 106 can be modified to provide an aspiration crack pressure of between 3 psi and 4psi. However, it will be appreciated that greater or lesser crack pressures are also contemplated.

While a change in radius of curvature (r) can modify a crack pressure on the convex side, which as shown in FIG. 2C, may be for aspiration, the change in radius of curvature (r) can have a lesser effect on the crack pressure on the concave side, which as shown in FIG. 2C may be for infusion. Advantageously, the crack pressures for either aspiration or infusion can be modified independently of each other depending on the direction of curve and the radius of curvature (r) applied.

In an embodiment, valve 100 can include a curved transverse axis 108 that can vary in radius curvature (r), as described herein. In an embodiment, the valve 100 can be formed with a linear lateral axis 106 and a linear transverse axis 108 and then constrained to a curved shape along either of the lateral axis 106 or transverse axis 108. In an embodiment, the valve 100 can be formed to include a curved shape along either of the lateral axis 106 or transverse axis 108, such that the valve maintains the curved shape in a resting state.

Advantageously, the curved profile along either the lateral axis 106 or the transverse axis 108 provides uniform structural support across the valve 100 leading to more precise crack pressures. This contrasts with valves that are supported by structures such as arms, bars, posts, or the like, which provide uneven support across the valve and can affect the crack pressure and tolerance ranges thereof.

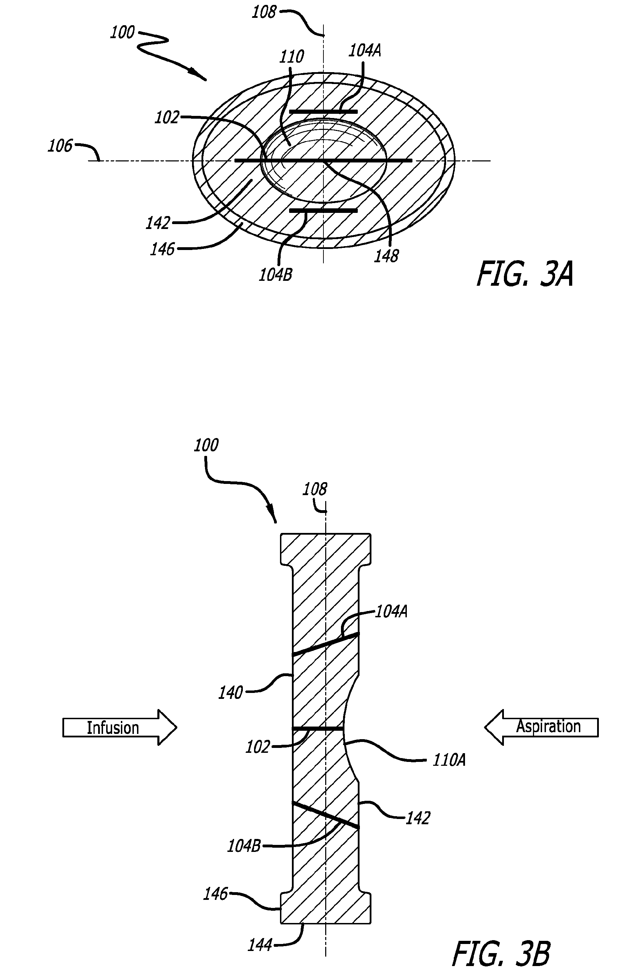

FIGS. 3A-3B show a distal end view and a cross-sectional view along a transverse axis 108 of an embodiment of a valve 100. In an embodiment, one of the proximal valve face 140 or the distal valve face 142 can include various reliefs that can modify the crack pressure of one or more of the slits 102, 104A, 104B. In an embodiment, the valve 100 can include a recess 110 disposed about a mid-point 148. The recess 110 can encircle a portion of one or more of the center slit 102, or the side slit(s) 104A, 104B. In an embodiment, the recess 110 can encircle the entire center slit 102. In an embodiment, the recess 110 can be disposed on the distal surface 142 and can be configured to provide a relatively high infusion crack pressure and a relatively low aspiration crack pressure.

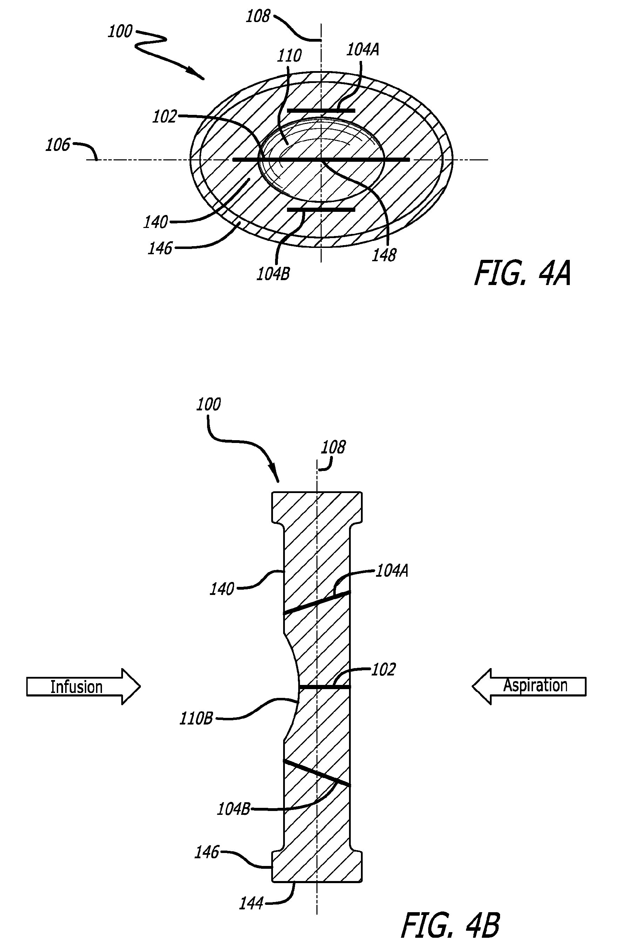

As shown in FIGS. 4A-4B, in an embodiment the recess 110 can be disposed on the proximal surface 140 and can be configured to provide a relatively high aspiration crack pressure and a relatively low infusion crack pressure. In an embodiment, the valve can include a first recess 110A disposed on a distal surface and a second recess 110B disposed on a proximal surface. In an embodiment the recesses 110A, 110B can be the same. In an embodiment the recesses 110A, 110B can be different. In an embodiment, the longitudinal depth, the lateral axis diameter, the transverse axis diameter, or radius of curvature of either of the recesses 110A, 110B can be varied to further modify the crack pressure of the portion of slit, e.g. slit 102, disposed therein. In an embodiment, a difference in ratio between the portion of slit 102, 104 disposed within the recess compared with the portion of slit 102, 104 disposed outside the recess can be modified to change the crack pressure of the overall slit 102, 104.

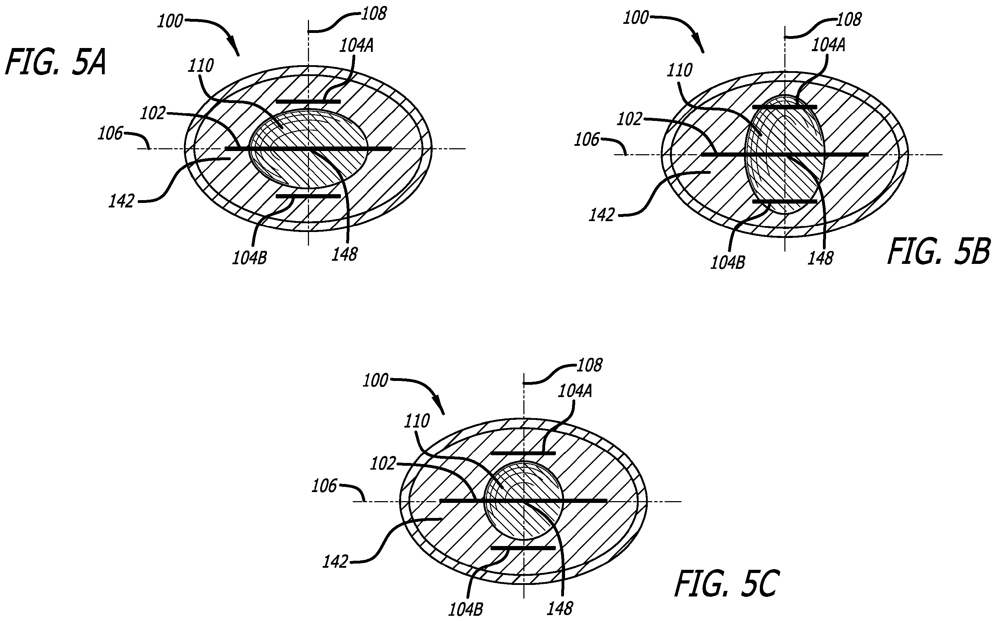

As shown in FIG. 5A, in an embodiment, the lateral axis diameter of the recess 110 can be longer than a transverse axis diameter of the recess 110 to provide an elliptical outer perimeter of the recess 110 that substantially matches the outer perimeter of the proximal face 140 or distal face 142 of the valve 100. As shown in FIG. 5B, in an embodiment, the lateral axis diameter of the recess 110 can be shorted than a transverse axis diameter of the recess 110 to provide an elliptical outer perimeter that is oriented at 90° relative to the outer perimeter of the proximal face 140 or distal face 142 of the valve 100. As shown in FIG. 5C, in an embodiment, the lateral axis diameter of the recess 110 can the same as the transverse axis diameter of the recess 110 to provide circular outer perimeter of the recess 110. As will be appreciated, various cross-sectional shapes of recess 110, in addition to elliptical and circular, are also contemplated and can also be configured to modify crack pressure of a portion of a slit disposed therein, e.g. slit 102. For example, other cross-sectional shapes of recess 110 can include oblong, square, rectangular, hexagonal, or any closed curve regular or irregular polygonal shape.

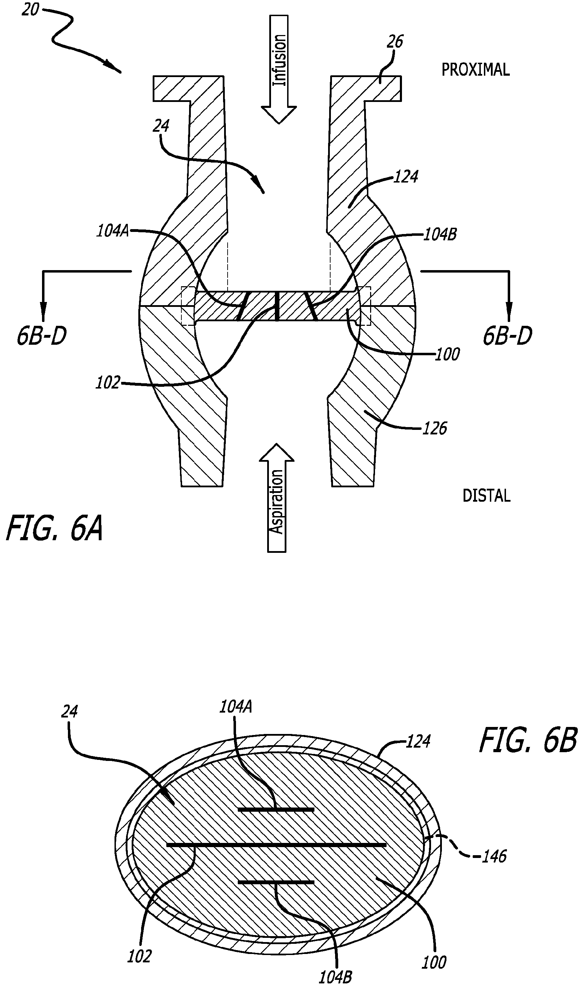

As shown in FIGS. 1B and 6A, in an embodiment, the valve 100 can be retained within the connector body 22. FIG. 6A shows a cross-sectional view of the connector body 22 including the valve 100 retained therein. The connector body 22 can be formed of a proximal housing piece 124 and a distal housing piece 126 that engage along a plane extending perpendicular to the longitudinal axis. The proximal housing piece 124 and the distal housing piece 126 may be assembled using a joining process such as solvent bonding, ultrasonic welding, adhesive bonding, or the like. The proximal housing piece 124 and the distal housing piece 126 can retain a portion of the valve 100 therebetween, for example rim 146.

Advantageously, retaining the valve 100 between the proximal housing piece 124 and the distal housing piece 126 allows for a higher precision in compression of the valve when retained by the connector 20. Differences in valve compression can affect crack pressure or maintenance pressure of either infusion or aspiration, tolerances thereof, or similar specifications. As such, assembling valve 100 and connector body 20 in this manner allows for precise performance characteristics of the valve 100, as well as consistency in valve performance. Further, this method of manufacture also allows the valve 100 to be assembled into the connector body 22, after the connector 20 has been molded. This mitigates damage to the valve 100 during manufacture, for example by eliminating the need for molding core pins to pass through the valve 100 and cause damage.

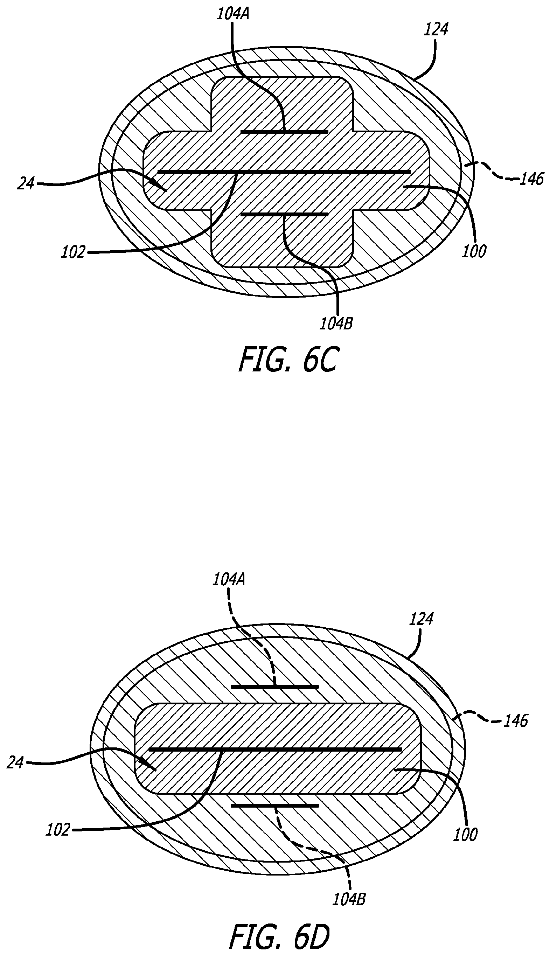

FIGS. 6B-6D illustrate a proximal end view of a cross-section of embodiments of the proximal housing piece 124 of FIG. 6A. As shown, a cross-sectional shape of the connector lumen 24 can define various closed curve, regular or irregular polygonal shapes. For example, the lumen 24 can define a substantially oval shape (FIG. 6B), a cross-shape shape (FIG. 6C), an oblong (FIG. 6D), or the like, without limitation. It is important to note, however, that the configuration of the lumen 24 can provide different cross-sectional surface areas. A relatively large cross-sectional area of the lumen 24 of the proximal housing 124 (e.g. FIG. 6B) can provide a relatively low aspiration crack pressure. By contrast, a relatively small cross-sectional area of the lumen 24 of the proximal housing 124 (e.g. FIG. 6D) can provide a relatively high aspiration crack pressure for the valve 100. In like manner, a cross-sectional shape and thereby a cross-sectional area of the lumen 24 of the distal housing piece 126 can also be modified to modify the infusion crack pressure of the valve 100. For example, a relatively small cross-sectional area of the lumen 24 of the distal housing 126 (e.g. FIG. 6D) can provide a relatively high infusion crack pressure for the valve 100.

In an embodiment, the cross-sectional shape can be configured to direct a fluid flow towards a center slit 102, or a side slit 104, or combinations thereof. For example, the cross-shaped cross-sectional area of the proximal housing 124 shown in FIG. 6C can direct an infusion flow to the center slit 102, as well as the side slits 104A, 104B. This can cause all slits 102, 104A, 104B, to open during infusion. By contrast, the oblong shaped cross-sectional area of the proximal housing 124 shown in FIG. 6D can direct an infusion flow to the center slit 102 only. This can cause only the center slit 102 to open during infusion. In like manner, a cross-sectional shape of the lumen of the distal housing 126 can be similarly modified to affect slit actuation during aspiration. These and other configurations of lumen shape of the proximal housing are also contemplated.

Advantageously, the design of the proximal housing piece 124 and the distal housing piece 126, can be modified to achieve a precise slit crack pressures for either infusion or aspiration. This, together with embodiments of the valve 100 as described herein, allow the valve 100 to be manufactured to a variety of specifications and within a precise range tolerances. Further the design of the connector body 20 and the valve can provide a turbulent flow through the connector lumen resulting in improved clearance characteristics. In addition, the design of the connector body 20 and valve 100, as described herein can provide a high crack pressure but also a low maintenance pressure. This reduces the pressure drop across the valve during use and can mitigate blood hemolysis. Advantageously, embodiments disclosed herein provide valves that can be manufactured to precise specifications in crack pressure or maintenance pressure for either infusion or aspiration. The specifications can be tuned to mitigate blood hemolysis and improve clearance characteristics. Further, the precise valve specifications can be easily achieved by modifying the valve 100 or the connector body housings 124, 126, as described herein, during manufacture. This results in improved manufacturing efficiencies and associated cost savings.

The valves disclosed herein can be molded in a single piece from an elastomeric material. Exemplary elastomeric materials can include a silicone rubber, or similar material, having a Shore A Durometer rating from about 30 to 60. Exemplary elastomeric materials can also include, without limitation, polyisoprene, butyl rubber, halogenated butyl rubbers, polybutadiene, styrene-butadiene rubber, nitrile rubber, hydrated nitrile rubbers, Therban^{®} elastomer, Zetpol^{®} elastomer, chloroprene rubber, polychloroprene, neoprene, baypren, EPM (ethylene propylene rubber), EPDM rubber (ethylene propylene diene rubber), epichlorohydrin rubber, polyacrylic rubber, silicone rubber, fluorosilicone rubber, fluoroelastomers, Viton^{®} elastomer, Tecnoflon^{®} elastomer, Fluorel^{®} elastomer, Dai-El^{®} elastomer, perfluoroelastomers, tetrafluoro ethylene/propylene rubbers, chlorosulfonated polyethylene, Hypalon^{®} elastomer, ethylene-vinyl acetate, Hytrel^{®} elastomer, Santoprene^{®} elastomer, polyurethane rubber, resilin, elastin, or Polysulfide rubber.

The connector housing pieces discussed herein can be molded in one or more pieces from a substantially rigid material. Exemplary materials can include a thermoplastic material having a Shore A Durometer rating from about 60 to 85. Further exemplary materials can include, without limitation, poly-ethylene terephthalate, IsoPlast^{®}, acrylonitrile butadiene styrene, acrylic, celluloid, cellulose acetate, ethylene-vinyl acetate, ethylene vinyl alcohol, fluoroplastics, ionomers, polyacetal, polyacrylates, polyacrylonitrile, polyamide, polyamideimide polyaryletherketone, polybutadiene, polybutylene, polybutylene terephthalate, polyethylene terephthalate, polycyclohexylene dimethylene terephthalate, polycarbonate, polyhydroxyalkanoates, polyketone, polyester, polyethylene, polyetheretherketone, polyetherimide, polyethersulfone, polyethylenechlorinates, polyimide, polylactic acid, polymethylpentene, polyphenylene oxide, polyphenylene sulfide, polyphthalamide, polypropylene, polystyrene, polysulfone, or polyvinyl chloride.

Any of the exemplary catheters described herein can be manufactured from any biocompatible material suitable for placement subcutaneously into a patient.

While some particular embodiments have been disclosed herein, and while the particular embodiments have been disclosed in some detail, it is not the intention for the particular embodiments to limit the scope of the concepts provided herein. The invention is defined by the appended claims.

## Claims

1. A valved connector (20), comprising:
a connector body (22) defining a lumen (24); and
a valve (100) configured to control a fluid flow through the lumen (24), the valve (100) including a proximal face (140) and a distal face (142), one of the proximal face (140) or the distal face (142) defining an oval shape, the valve (100) defining a curved shape through a lateral axis (106) and extend linearly through a transverse axis (108), the valve (100) including a slit (102, 104A, 104B) extending from the proximal face (140) to the distal face (142),
wherein a longitudinal axis extends substantially parallel to an axial length of the valved connector (20), the lateral axis (106) extends normal to the longitudinal axis, and the transverse axis (108) extends normal to both the longitudinal and lateral axes; and
wherein the oval shape includes a widest diameter extending along the lateral axis (106), and a shortest diameter extending along the transverse axis (108).

2. The valved connector (20) according to claim 1, further including a center slit (102) and a side slit (104A, 104B) each extending parallel to the lateral axis (106), the side slit (104A, 104B) disposed in an off-set relationship along the transverse axis (108) from the center slit (102).

3. The valved connector (20) according to claim 2, wherein the side slit (104A, 104B) extends through the valve (100) from the proximal face (140) to the distal face (142) at an angle relative to the longitudinal axis.

4. The valved connector according to claim 3, wherein a first side slit (104A) is angled in a first direction relative to the longitudinal axis and a second side slit (104B) is angled in a second direction opposite the first direction, relative to the longitudinal axis.

5. The valved connector (20) according to claim 2, wherein the center slit (102) is configured to open during both infusion and aspiration and the side slit (104A, 104B) is configured to open only during infusion.

6. The valved connector according to any of the preceding claims, wherein one of the proximal face (140) or the distal face (142) of the valve (100) includes a recess (110) encircling a portion of the slit (102, 104A, 104B).

7. The valved connector (20) according to any of the preceding claims, wherein the connector body (22) includes a proximal housing piece (124) defining a first lumen and a distal housing piece (126) defining a second lumen, and wherein a portion of the valve (100) is retained between the proximal housing piece (124) and the second housing piece to control a fluid flow between the first lumen and the second lumen.

8. The valved connector (20) according to claim 7, wherein a portion of the first lumen defines a reduced cross-sectional area to modify an aspiration crack pressure.

9. The valved connector according to claim 7, wherein a portion of the first lumen defines one of an oval shape, an oblong shape or a cross shape to direct a fluid flow towards the slit (102, 104A, 104B).

10. A method of manufacturing the valved connector (20) in accordance with any of claims 1-9, comprising:
forming a proximal housing piece (124) including a first lumen and a distal engagement surface;
forming a distal housing piece (126) including a second lumen and a proximal engagement surface;
forming a valve (100) including a proximal face (140) and a distal face (142) and a slit (102, 104A, 104B) extending therebetween, one of the proximal face (140) or the distal face (142) defining an oval shape, a lateral axis (106) of the oval shape being wider than a transverse axis (108) of the oval shape;
retaining the valve (100) between the proximal housing piece (124) and the distal housing piece (126) to control a fluid flow between the first lumen and the second lumen;
constraining the lateral axis (106) of the valve (100) to a curved shape to provide a convex shape to the proximal face (140); and
attaching the distal engagement surface with the proximal engagement surface,
wherein a longitudinal axis extends substantially parallel to an axial length of the valved connector (20), the lateral axis (106) extends normal to the longitudinal axis, and the transverse axis (108) extending normal to both the longitudinal and lateral axes.

## Patentansprüche

1. Mit Ventil versehener Verbinder (20), umfassend:
einen Verbinderkörper (22), der ein Lumen (24) definiert; und
ein Ventil (100), das dazu konfiguriert ist, einen Fluidstrom durch das Lumen (24) zu steuern, wobei das Ventil (100) eine proximale Fläche (140) und eine distale Fläche (142) einschließt, wobei eine der proximalen Fläche (140) oder der distalen Fläche (142) eine ovale Form definiert, wobei das Ventil (100) eine gekrümmte Form durch eine laterale Achse (106) definiert und sich linear durch eine transversale Achse (108) erstreckt, wobei das Ventil (100) einen Schlitz (102, 104A, 104B) einschließt, der sich von der proximalen Fläche (140) zu der distalen Fläche (142) erstreckt,
wobei sich eine longitudinale Achse im Wesentlichen parallel zu einer axialen Länge des mit Ventil versehenen Verbinders (20) erstreckt, sich die laterale Achse (106) normal zu der longitudinalen Achse erstreckt und sich die transversale Achse (108) normal sowohl zu der longitudinalen als auch zu der lateralen Achse erstreckt; und
wobei die ovale Form einen breitesten Durchmesser, der sich entlang der lateralen Achse (106) erstreckt, und einen kürzesten Durchmesser, der sich entlang der transversalen Achse (108) erstreckt, einschließt.

2. Mit Ventil versehener Verbinder (20) nach Anspruch 1, weiter einschließend einen zentralen Schlitz (102) und einen seitlichen Schlitz (104A, 104B), die sich jeweils parallel zu der lateralen Achse (106) erstrecken, wobei der seitliche Schlitz (104A, 104B) in einer versetzten Beziehung entlang der transversalen Achse (108) von dem zentralen Schlitz (102) angeordnet ist.

3. Mit Ventil versehener Verbinder (20) nach Anspruch 2, wobei sich der seitliche Schlitz (104A, 104B) durch das Ventil (100) von der proximalen Fläche (140) zu der distalen Fläche (142) in einem Winkel relativ zu der longitudinalen Achse erstreckt.

4. Mit Ventil versehener Verbinder nach Anspruch 3, wobei ein erster seitlicher Schlitz (104A) in einer ersten Richtung relativ zu der longitudinalen Achse abgewinkelt ist und ein zweiter seitlicher Schlitz (104B) in einer zweiten Richtung, die der ersten Richtung entgegengesetzt ist, relativ zu der longitudinalen Achse abgewinkelt ist.

5. Mit Ventil versehener Verbinder (20) nach Anspruch 2, wobei der zentrale Schlitz (102) dazu konfiguriert ist, sich sowohl während Infusion als auch während Aspiration zu öffnen, und der seitliche Schlitz (104A, 104B) dazu konfiguriert ist, sich nur während Infusion zu öffnen.

6. Mit Ventil versehener Verbinder nach einem der vorstehenden Ansprüche, wobei eine der proximalen Fläche (140) oder der distalen Fläche (142) des Ventils (100) eine Aussparung (110) einschließt, die einen Abschnitt des Schlitzes (102, 104A, 104B) einkreist.

7. Mit Ventil versehener Verbinder (20) nach einem der vorstehenden Ansprüche, wobei der Verbinderkörper (22) ein proximales Gehäusestück (124), das ein erstes Lumen definiert, und ein distales Gehäusestück (126), das ein zweites Lumen definiert, einschließt, und wobei ein Abschnitt des Ventils (100) zwischen dem proximalen Gehäusestück (124) und dem distalen Gehäusestück zurückgehalten wird, um einen Fluidstrom zwischen dem ersten Lumen und dem zweiten Lumen zu steuern.

8. Mit Ventil versehener Verbinder (20) nach Anspruch 7, wobei ein Abschnitt des ersten Lumens einen reduzierten Querschnittsbereich definiert, um einen Aspirationsrissdruck zu modifizieren.

9. Mit Ventil versehener Verbinder nach Anspruch 7, wobei ein Abschnitt des ersten Lumens eine von einer ovalen Form, einer länglichen Form oder einer kreuzförmigen Form definiert, um einen Fluidstrom zu dem Schlitz (102, 104A, 104B) hin zu richten.

10. Verfahren zum Herstellen des mit Ventil versehenen Verbinders (20) nach einem der Ansprüche 1-9, umfassend:
Bilden eines proximalen Gehäusestücks (124), das ein erstes Lumen und eine distale Eingriffsfläche einschließt;
Bilden eines distalen Gehäusestücks (126), das ein zweites Lumen und eine proximale Eingriffsfläche einschließt;
Bilden eines Ventils (100), das eine proximale Fläche (140) und eine distale Fläche (142) und einen Schlitz (102, 104A, 104B), der sich dazwischen erstreckt, einschließt, wobei eine der proximalen Fläche (140) oder der distalen Fläche (142) eine ovale Form definiert, wobei eine laterale Achse (106) der ovalen Form breiter als eine transversale Achse (108) der ovalen Form ist;
Zurückhalten des Ventils (100) zwischen dem proximalen Gehäusestück (124) und dem distalen Gehäusestück (126), um einen Fluidstrom zwischen dem ersten Lumen und dem zweiten Lumen zu steuern;
Beschränken der lateralen Achse (106) des Ventils (100) auf eine gekrümmte Form, um der proximalen Fläche (140) eine konvexe Form bereitzustellen; und
Anbringen der distalen Eingriffsfläche an der proximalen Eingriffsfläche,
wobei sich eine longitudinale Achse im Wesentlichen parallel zu einer axialen Länge des mit Ventil versehenen Verbinders (20) erstreckt, sich die laterale Achse (106) normal zu der longitudinalen Achse erstreckt und sich die transversale Achse (108) normal sowohl zu der longitudinalen als auch der lateralen Achse erstreckt.

## Revendications

1. Raccord à vanne (20), comprenant :
un corps de raccord (22) définissant une lumière (24) ; et
une vanne (100) configurée pour commander un écoulement de fluide à travers la lumière (24), la vanne (100) incluant une face proximale (140) et une face distale (142), l'une de la face proximale (140) ou de la face distale (142) définissant une forme ovale, la vanne (100) définissant une forme incurvée à travers un axe latéral (106) et s'étendant linéairement à travers un axe transversal (108), la vanne (100) incluant une fente (102, 104A, 104B) s'étendant de la face proximale (140) à la face distale (142),
dans lequel un axe longitudinal s'étend sensiblement parallèlement à une longueur axiale du raccord à vanne (20), l'axe latéral (106) s'étend perpendiculairement à l'axe longitudinal, et l'axe transversal (108) s'étend perpendiculairement à la fois aux axes longitudinal et latéral ; et
dans lequel la forme ovale inclut un diamètre le plus large s'étendant le long de l'axe latéral (106), et un diamètre le plus court s'étendant le long de l'axe transversal (108).

2. Raccord à vanne (20) selon la revendication 1, incluant en outre une fente centrale (102) et une fente latérale (104A, 104B) s'étendant chacune parallèlement à l'axe latéral (106), la fente latérale (104A, 104B) étant disposée dans une relation décalée le long de l'axe transversal (108) par rapport à la fente centrale (102).

3. Raccord à vanne (20) selon la revendication 2, dans lequel la fente latérale (104A, 104B) s'étend à travers la vanne (100) de la face proximale (140) à la face distale (142) selon un angle par rapport à l'axe longitudinal.

4. Raccord à vanne selon la revendication 3, dans lequel une première fente latérale (104A) est inclinée dans une première direction par rapport à l'axe longitudinal et une seconde fente latérale (104B) est inclinée dans une seconde direction opposée à la première direction, par rapport à l'axe longitudinal.

5. Raccord à vanne (20) selon la revendication 2, dans lequel la fente centrale (102) est configurée pour s'ouvrir à la fois pendant une perfusion et une aspiration et la fente latérale (104A, 104B) est configurée pour s'ouvrir uniquement pendant une perfusion.

6. Raccord à vanne selon l'une quelconque des revendications précédentes, dans lequel l'une de la face proximale (140) ou de la face distale (142) de la vanne (100) inclut un évidement (110) entourant une partie de la fente (102, 104A, 104B).

7. Raccord à vanne (20) selon l'une quelconque des revendications précédentes, dans lequel le corps de raccord (22) inclut une pièce de boîtier proximale (124) définissant une première lumière et une pièce de boîtier distale (126) définissant une seconde lumière, et dans lequel une partie de la vanne (100) est maintenue entre la pièce de boîtier proximale (124) et la seconde pièce de boîtier de manière à commander un écoulement de fluide entre la première lumière et la seconde lumière.

8. Raccord à vanne (20) selon la revendication 7, dans lequel une partie de la première lumière définit une zone de section transversale réduite pour modifier une pression d'ouverture à l'aspiration.

9. Raccord à vanne selon la revendication 7, dans lequel une partie de la première lumière définit l'une d'une forme ovale, d'une forme oblongue ou d'une forme en croix pour diriger un écoulement de fluide vers la fente (102, 104A, 104B).

10. Procédé de fabrication du raccord à vanne (20) selon l'une quelconque des revendications 1 à 9, comprenant :
la formation d'une pièce de boîtier proximale (124) incluant une première lumière et une surface de mise en prise distale ;
la formation d'une pièce de boîtier distale (126) incluant une seconde lumière et une surface de mise en prise proximale ;
la formation d'une vanne (100) incluant une face proximale (140) et une face distale (142) et une fente (102, 104A, 104B) s'étendant entre celles-ci, l'une de la face proximale (140) ou de la face distale (142) définissant une forme ovale, un axe latéral (106) de la forme ovale étant plus large qu'un axe transversal (108) de la forme ovale ;
le maintien de la vanne (100) entre la pièce de boîtier proximale (124) et la pièce de boîtier distale (126) de manière à commander un écoulement de fluide entre la première lumière et la seconde lumière ;
la mise en contrainte de l'axe latéral (106) de la vanne (100) dans une forme incurvée de manière à donner une forme convexe à la face proximale (140) ; et
la fixation de la surface de mise en prise distale à la surface de mise en prise proximale,
dans lequel un axe longitudinal s'étend sensiblement parallèlement à une longueur axiale du raccord à vanne (20), l'axe latéral (106) s'étend perpendiculairement à l'axe longitudinal, et l'axe transversal (108) s'étend perpendiculairement à la fois aux axes longitudinal et latéral.
